# EUROPEAN PATENT APPLICATION

(11) **EP 1 921 432 A1**
(43) Date of publication of application: **14.05.2008**
(21) Application number: 06781826.0
(22) Date of filing: 27.07.2006
(51) Int. Cl.: G01L 5/00, A61B 5/00, A61B 5/22

(54) **PROBE FOR MEASURING ORAL CAVITY-RELATED PRESSURE, DEVICE FOR MEASURING ORAL CAVITY-RELATED PRESSURE, AND TRAINING DEVICE FOR RECOVERING ORAL CAVITY FUNCTION**

(30) Priority: 31.08.2005 JP 2005251960
(71) Applicant: JMS Co., Ltd., Hiroshima-shi, Hiroshima 730-8652 (JP)
(72) Inventor: MIYAHARA, Hideyasu c/o JMS Co.Ldt, Hiroshima 730-8652 (JP); TOYOTA, Koichiro c/o JMS Co.Ldt, Hiroshima 730-8652 (JP)
(74) Representative: Stippl, Hubert
(86) International application number: PCT/JP2006/314915
(87) International publication number: WO 2007/026488

(57) **Abstract**

A probe for measuring oral pressure according to the present invention includes: a balloon 1; a hollow tube 3 that has a first opening portion 3b and a second opening portion 3c and is connected to the balloon so that the first opening potion 3b communicates with an inside of the balloon 1; and a port member 20 with a valve 13 connected to the hollow tube 3 so as to block the second opening portion 3c. The probe for measuring oral pressure according to the present invention preferably further includes a hard ring 8 mounted on a portion between an inflated portion and an attaching portion of the balloon 1. The valve 13 may be a disk-shaped valve with an insertion hole 13a, for example.

## Description

### Technical Field

The present invention relates to a probe for measuring oral pressure that is used to measure oral pressure such as lingual pressure, pressure of hypoglossal muscle, lip pressure, cheek pressure, and the like, and a device for measuring oral pressure using the same. The present invention further relates to a training tool used for restoring an oral function of a patient.

### Background Art

In recent years, for the improvement of the QOL (Quality of Life) of elderly people, a feeding and swallowing function needs to be maintained and restored. To this end, the elucidation of such a function has been required. The feeding and swallowing function closely is related with the movement of a tongue, and a predetermined lingual pressure is required to form a bolus and send it to a pharynx. For this reason, it is important to measure and analyze lingual pressure.

For example, a known method for measuring lingual pressure uses a balloon probe (for example, see Patent Documents 1 and 2). A measuring device disclosed in Patent Document 1 uses a balloon as a probe. When the balloon is inserted into an oral cavity to be pressed by a tongue, a change in air pressure in the balloon is detected by a conversion portion that communicates with the balloon. The change in air pressure is converted into an electric signal, thereby measuring lingual pressure. A measuring device disclosed in Patent Document 2 includes, as shown in FIG. 11, a disposable probe 110, a pressurizing portion 112 that communicates with the probe 110, a pressure detecting portion 117 for detecting air pressure in a balloon 111, and the like. When lingual pressure or the like of a patient is measured by using such measuring devices, it becomes possible to serve a meal according to the patient's condition, and accordingly the patient can be prevented from suffering from aspiration pneumonitis or the like, for example.
Patent Document 1: US Patent No. 5609161
Patent Document 2: JP 2001-275994 A

For the improvement of the QOL of elderly people and the like, it is desired to restore positively an oral function such as a lingual function, for example, as well as to serve an appropriate meal to a patient. The present inventors considered that the air pressure in the balloon can be used to restore a lingual function. However, the probe 110 described in Patent Document 2 cannot keep the balloon 111 inflated once it is disconnected from the pressurizing portion 112 such as an air pump, for example. Thus, in order for a patient to undergo rehabilitation for restoring a lingual function, the probe 110 has to be connected constantly to the pressurizing portion 112 or the like, which is inconvenient and costly. For this reason, it is practically difficult to undergo rehabilitation using the probe 110 at home.

The present invention provides a probe for measuring oral pressure that also can be used easily as a rehabilitation tool for restoring an oral function.

### Disclosure of Invention

A probe for measuring oral pressure according to the present invention includes: a balloon; a hollow tube that has a first opening portion and a second opening portion and is connected to the balloon so that the first opening potion communicates with an inside of the balloon; and a port member with a valve connected to the hollow tube so as to block the second opening portion.

A device for measuring oral pressure according to the present invention includes: the probe for measuring oral pressure according to the present invention; and a pressure detecting portion that communicates with an inside of the balloon to detect air pressure in the balloon.

A training tool for restoring an oral function according to the present invention includes: a balloon; a hollow tube that has a first opening portion and a second opening portion and is connected to the balloon so that the first opening potion communicates with an inside of the balloon; and a port member with a valve connected so as to block the second opening portion.

### Brief Description of Drawings

[FIG. 1] FIG. 1 is a perspective view showing an example of a probe for measuring oral pressure according to Embodiment 1.
[FIG. 2] FIG. 2 is another perspective view of the probe for measuring oral pressure shown in FIG. 1.
[FIG. 3] FIG. 3 is a cross-sectional view of the probe for measuring oral pressure shown in FIG. 1, taken along a line I-I.
[FIG. 4A] FIG. 4A is an enlarged cross-sectional view for explaining a port member constituting the probe for measuring oral pressure shown in FIG. 1.
[FIG. 4B] FIG. 4B is an enlarged cross-sectional view for explaining a state where an insertion member is inserted into an insertion hole constituting the probe for measuring oral pressure shown in FIG. 1.
[FIG. 5] FIG. 5 is a side view of the probe for measuring oral pressure shown in FIG. 1, seen from a valve side.
[FIG. 6] FIG. 6 is a perspective view showing another example of the probe for measuring oral pressure according to Embodiment 1.
[FIG. 7] FIG. 7 is a perspective view showing still another example of the probe for measuring oral pressure according to Embodiment 1.
[FIG. 8] FIG. 8 is a perspective view showing still another example of the probe for measuring oral pressure according to Embodiment 1.
[FIG. 9] FIG. 9 is a conceptual diagram showing an example of a device for measuring oral pressure according to Embodiment 2.
[FIG. 10] FIG. 10 is a conceptual diagram showing a condition of use of a probe for measuring oral pressure constituting the device for measuring oral pressure shown in FIG. 9.

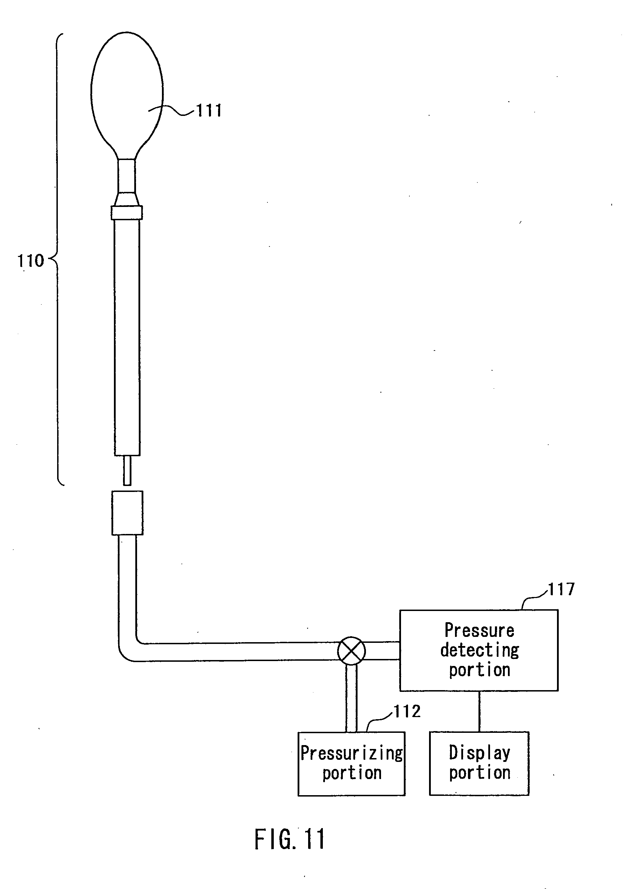

[FIG. 11] FIG. 11 is a conceptual diagram showing an example of a conventional device for measuring oral pressure.

### Explanation of Letters or Numerals

- 1: Balloon

- 1a: Inflated portion
- 1b: Attaching portion
- 3: Hollow tube
- 3b: First opening portion
- 3c: Second opening portion
- 4: First connecting portion
- 5: Second connecting portion
- 6: Main body tube
- 7: Pressure detecting portion
- 8: Hard ring
- 9: Display portion
- 10: Probe for measuring oral pressure
- 11: Selector valve
- 13: Valve
- 13a: Insertion hole (slit)
- 14: Bumper
- 14a: Through hole
- 16: Cover
- 16a: Protruding portion
- 16b: Fitting hole
- 16c: Circular hook portion
- 100: Device for measuring oral pressure

### Description of the Invention

In an example of a probe for measuring oral pressure (hereinafter, this may be abbreviated as a "probe") according to the present invention, the valve may be a rubber stopper or the like, for example, and also may be a valve with a slit. The valve is disk-shaped, for example. Preferably, a male luer can be inserted into the slit of the valve. The male luer as used herein refers to an insertion member that tapers toward its end, and preferably is in conformity with the international standard (ISO594-1)

In an example of the probe of the present invention, the hollow tube may be bent into an L-shape, for example.

In an example of the probe of the present invention, preferably, the balloon has an inflated portion and an attaching portion to the hollow tube, and a hard ring is mounted on a portion between the inflated portion and the attaching portion of the balloon.

In an example of the probe of the present invention, the hard ring preferably has a flat, e.g., oval outer shape when seen in a longitudinal direction so that the hard ring can be seen internally.

In an example of the probe of the present invention, the inflated portion may have a spherical shape, for example, and preferably has a flat shape when the balloon is inflated.

In an example of the probe of the present invention, preferably, a bumper arranged on a side of the hollow tube rather than the inflated portion of the balloon further is provided. The bumper is arranged on an outer periphery of the hollow tube or the hard ring, for example. In the present application, the bumper serves as a stopper provided to prevent the probe from being inserted excessively into an oral cavity.

In an example of the probe of the present invention, the bumper preferably has a through hole that penetrates in a thickness direction (the same direction as a longitudinal direction of the probe) of the bumper. The bumper may be slidable in the longitudinal direction of the probe for measuring oral pressure.

The bumper preferably is made of an elastic material or a flexible material since it may be pressed against lips or the like. For the same reason, the bumper preferably can be bent under pressure applied from a balloon side.

In an example of the probe of the present invention, for example, the hollow tube may be calibrated for indicating a distance from a balloon side end of the probe.

It is also possible to provide a device for measuring oral pressure by using an example of the probe of the present invention. The device for measuring oral pressure includes: an example of the probe according to the present invention; and a pressure detecting portion that communicates with an inside of the balloon to detect air pressure in the balloon. An example of the device for measuring oral pressure includes a main body tube with two ends, one being connected to the pressure detecting portion and the other being connected to the probe for measuring oral pressure, that allows the inside of the balloon and the pressure detecting portion to communicate with each other. The pressure detecting portion and the main body tube are connected directly or indirectly. The hollow tube of the probe for measuring oral pressure and the main body tube each have connecting portions that can be connected attachably/detachably to each other. The probe and the main body tube preferably are connected by a luer lock system, for example, which allows airtight connection easily.

Next, an example of the present invention will be described in more detail with reference to the drawings.

### (Embodiment 1)

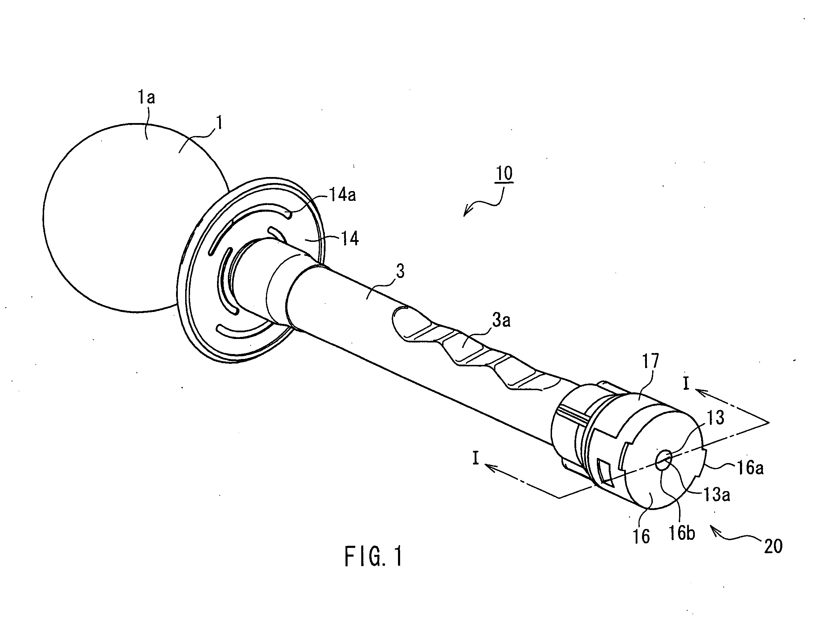

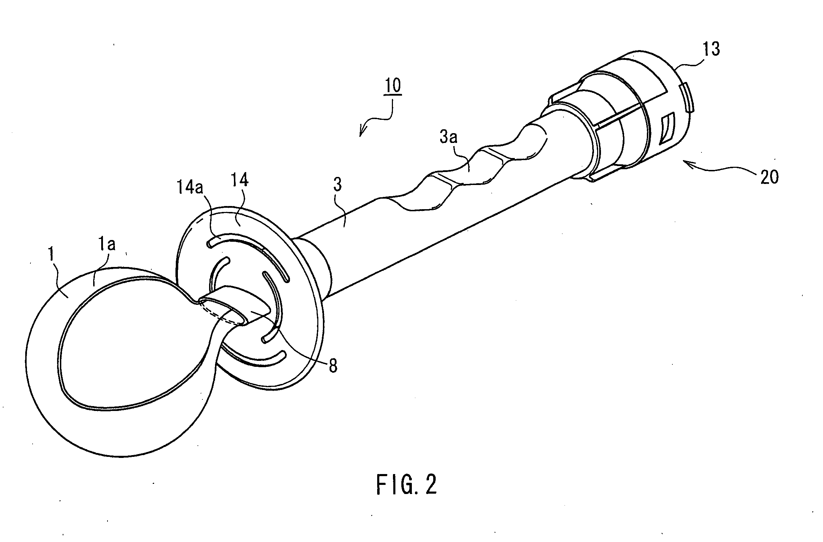

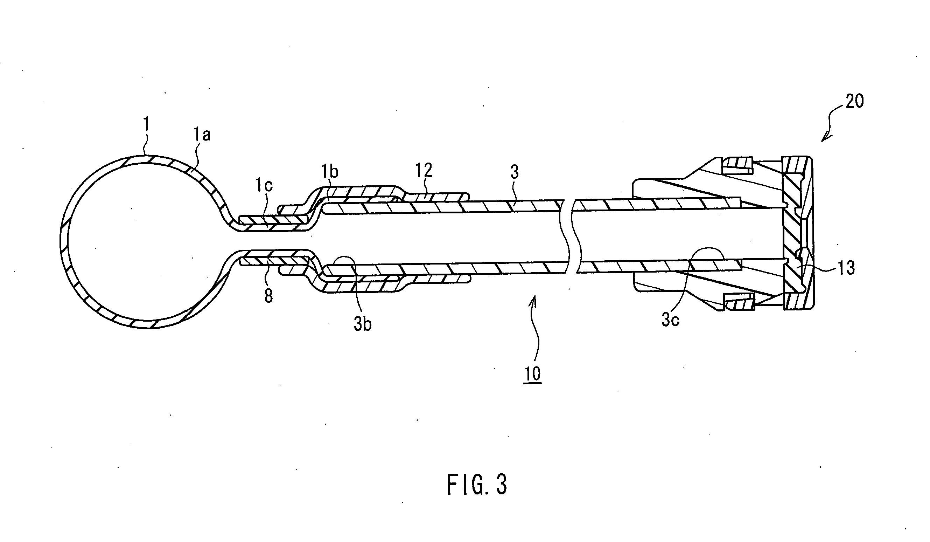

In Embodiment 1, an example of a probe for measuring oral pressure according to the present invention will be described. FIGs. 1 and 2 are perspective views showing an example of the probe for measuring oral pressure according to the present embodiment, and FIG. 3 is a cross-sectional view of the probe for measuring oral pressure shown in FIG. 1, taken along a line I-I. In FIG. 2, a balloon 1 partially is cut away so that a hard ring 8 can be seen easily. In FIG. 3, the bumper14 in FIGs. 1 and 2 is omitted.

As shown in FIGs. 1 and 2, a probe 10 includes the balloon 1, a hollow tube 3, and a port member 20. One opening portion (a first opening portion 3b, see FIG. 3) of the hollow tube 3 is connected with the balloon 1 so that the inside of the balloon 1 communicates with the hollow tube 3. An opening portion (a second opening portion 3c, see FIG. 3) of the hollow tube 3 on a side opposite to the balloon 1 side is connected with the port member 20. This allows a sealed space to be present in the probe 10. The port member 20 includes a valve 13, only through which gas can come in and out of the probe 10.

In the probe 10 of this embodiment, as shown in FIG. 3, the balloon 1 is connected directly to the hollow tube 3, and an attaching portion 1b of the balloon 1 is fixed airtightly to the hollow tube 3. The attaching portion 1b of the balloon 1 is tightened around the hollow tube 3 by the shrinkage of a heat shrinkable tube 12. The shrinkage of the heat shrinkable tube 12 allows the balloon 1 and the hollow tube 3 to be connected airtightly to each other.

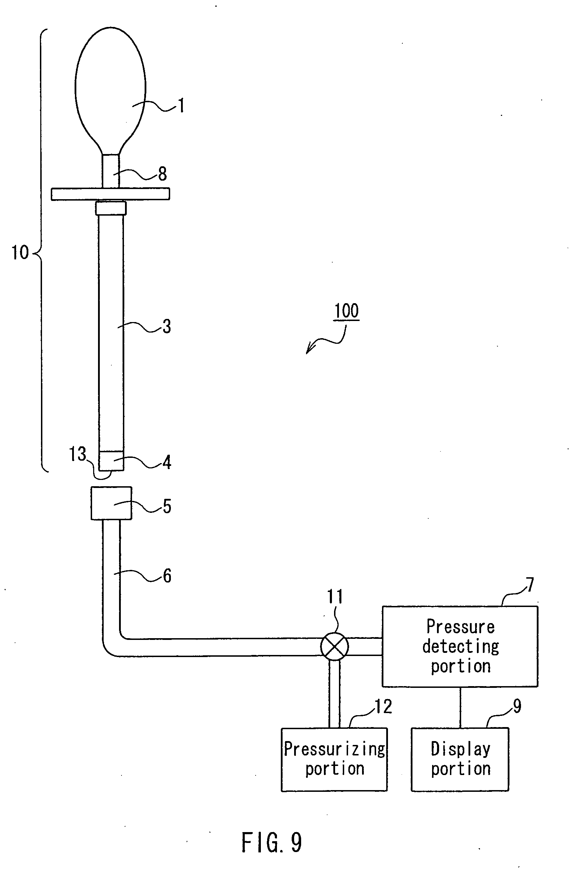

When lingual pressure is measured by using the probe 10 of this embodiment, as shown in FIG. 9, a selector valve 11 of a device 100 for measuring oral pressure is opened, and air or the like is supplied into the balloon 1 by a pressurizing portion 12 so that a predetermined pressure is present in the balloon 1. The balloon 1 pressurized to be inflated is put into a mouth, thereby measuring maximum lingual pressure, lingual pressure for swallowing, or the like. As shown in FIGs. 1 and 2, since the probe 10 of this embodiment includes the valve 13, the balloon 1 easily can be kept inflated even when the probe is disconnected from the pressurizing portion 12 (see FIG. 9) or the like. The probe 10 with the inflated balloon 1 can be used for training for increased lingual pressure. Thus, the probe 10 can be used not only for the measurement of lingual pressure but also as a training tool for restoring an oral function such as a lingual function.

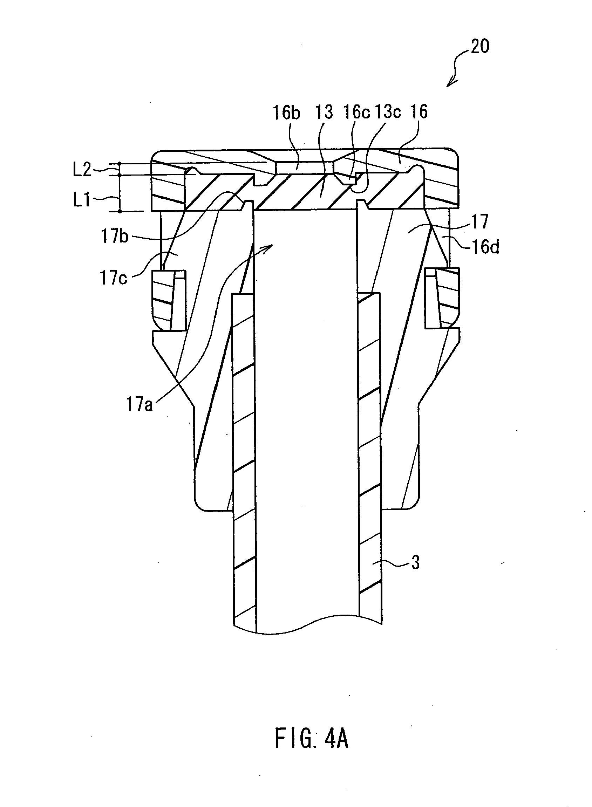

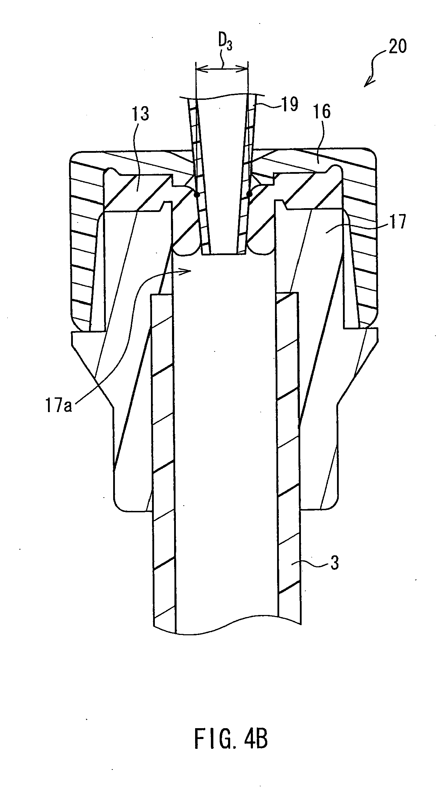

As shown in FIG. 4A, the valve 13 preferably is a disk-shaped valve with an insertion hole 13a (see FIG. 1) formed at its center, for example. In the case where the valve 13 is disk-shaped, for example, when an end portion of a needleless syringe (a male luer 19) (see FIG. 4B), for example, is inserted into the insertion hole 13a, it is possible to let air in and out of the balloon 1.

In the example shown in FIG. 4A, the port member 20 includes the valve 13, a pedestal 17, and a cover 16. The valve 13 is fixed to the hollow tube 3 via the pedestal 17 that supports the valve 13 in contact with a rear surface of the valve 13. The pedestal 17 has a hole 17a that allows the insertion member 19 (for example, a male luer, see FIG. 4B) and the hollow tube 3 to communicate internally with each other when the insertion member is inserted into the insertion hole 13a (see FIG, 1). The valve 13 has its outer peripheral surface and its side surface covered with the cover 16 that holds the valve 13 tightly.

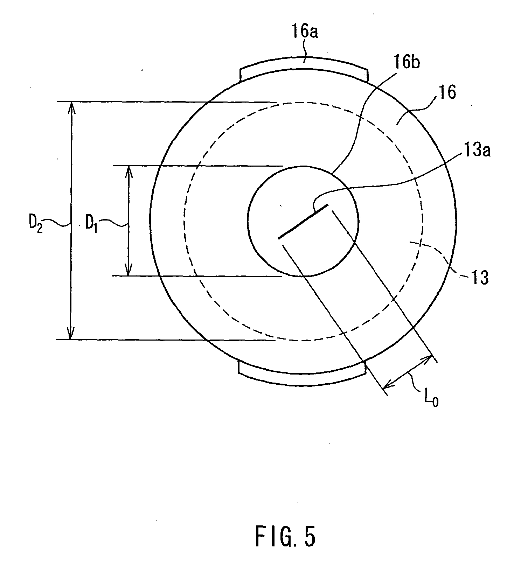

The valve 13 preferably has a circular or oval flat surface, for example. The insertion hole 13a may be formed of a slit-shaped line or three linear slits that intersect at the center, for example. However, as shown in FIG. 5, it is preferable for the sake of convenience that the insertion hole 13a is formed as a linear slit. In the case where the insertion member 19 (see FIG. 4B) is a general male luer, the slit 13a preferably has a length Lo of 3.0 to 4.5 mm in terms of the insertion capability and liquid-tightness of the valve 13.

As shown in FIG. 5, the ratio between an outer diameter D₂ of the valve 13 and the length L₀ of the slit preferably satisfies 1.1 ≤ D₂/L₀ ≤ 4 for the reason described below.

As shown in FIG. 4A, the valve 13 preferably has a thickness L₁ of 1 to 2 mm in terms of the non-return effect, cost efficiency, and the like. The valve 13 may be made of a rubber-like elastic material. More restrictively, a material with a hardness JIS-A of 20 to 55 is preferable. Specific examples of the material include a synthetic rubber such as a silicone rubber, a natural rubber, a butyl rubber, and a nitrile rubber, a thermoplastic elastomer, and the like.

The pedestal 17 preferably is provided with a circular rib 17b formed circularly around a peripheral portion of the hole 17a. The circular rib 17b is formed on a surface of the pedestal 17 that is in contact with the valve 13. In this manner, when the pedestal 17 includes the circular rib 17b, it is possible to prevent liquid leakage between the valve 13 and the pedestal 17 even if the valve 13 is deformed when the insertion member 19 (see FIG 4B) is inserted into the slit 13a of the valve 13 to communicate with the hollow tube 3.

The insertion member 19 (see FIG. 4B) inserted into the slit 13a can be engaged with the cover 16 by being fitted in a fitting hole 16b formed at the center of the cover 16, for example. In this case, the insertion member 19 can be engaged with the probe 10 with a simple configuration.

In the case where the insertion member 19 is a male luer with a 6/100 tapered surface as defined by the international standard (ISO594-1), the fitting hole 16b preferably has a diameter of 3.9 to 4.4 mm and a depth L₂ of 0.3 to 1.0 mm.

The cover 16 preferably has a sufficient strength so as not to be cracked even when the insertion member 19 is fitted tightly in the fitting hole 16b. On this account, the cover 16 preferably is made of polyacetal, polypropylene, polyamide, polyethylene terephthalate, polybutylene terephthalate, or the like, for example.

The valve 13 preferably has a circular cut-away portion 13c on its outer surface. The cut-away portion 13c is engaged with a circular hook portion 16c of the cover 16, and the valve 13 is sandwiched tightly between the circular hook portion 16c and the circular rib 17b, whereby the valve 13 is divided into a portion to be extended and a compressed portion. More specifically, when the insertion member 19 (see FIG. 4B) is inserted into the insertion hole 13a, only a portion inside the cut-away portion 13c of the valve 13 is extended, and a portion (compressed portion) outside thereof maintains the airtightness between the cover 16 and the pedestal 17. The extended portion of the valve 13 easily can return to its original state when the insertion member 19 is extracted from the insertion hole 13a. Further, the insertion member can be inserted into the insertion hole 13a while being guided by the circular hook portion 16c. Thus, both ease of insertion and the fitting capability of the insertion member can be improved.

The cover 16 may be tapered (inclined) gently toward the fitting hole 16b. The cover 16 may have at least two cut-away portions 16d in its outer portion, which may be engaged with protrusion portions 17c provided in the pedestal 17.

As shown in FIG. 5, it is assumed that the fitting hole 16b has a diameter D₁, the valve 13 has the outer diameter D₂, and the slit as the insertion hole 13a has the length L₀. In this case, it is preferable that 1.1 ≤ D₂/L₀ ≤ 4 is satisfied in terms of ease of insertion of the insertion member into the insertion hole 13a, the non-return effect, and the like. When the length Lo of the insertion hole 13a is too long, i.e., D₂/L₀ is smaller than 1.1, it is feared that the valve is deformed and broken (torn) by inserting the insertion member into the insertion hole 13a. In addition, a peripheral portion of the valve that is deformable (when the insertion member is inserted into the insertion hole) becomes smaller with respect to the insertion hole 13a, resulting in difficulty in inserting the insertion member into the insertion hole 13a. On the other hand, when D₂/L₀ is larger than 4, it becomes easier to insert the insertion member into the valve. However, the valve, the cover 16, and the like become larger, resulting in a cost increase.

A description will be given of the relationship between the length Lo of the slit as the insertion hole 13a and the insertion member 19 (see FIGs. 4A and 4B). It is assumed that in a state where the insertion member is engaged with the fitting hole 16b, a maximum diameter of a portion of the insertion member 19 that is buried in the valve 13 in contact therewith is an insertion portion diameter D₃. In this case, the length Lo of the slit preferably is 0.7 times or more and 1.1 times or less the insertion portion diameter D₃. When Lo is smaller than this range, it becomes difficult to insert the insertion member. When Lo is larger than this range, air easily leaks from the insertion hole when the insertion member 19 is extracted from the insertion hole 13a.

The hollow tube 3 preferably is made of a hard material for easy holding when the balloon 1 is put in a mouth. A particularly preferable material is rigid plastic such as polypropylene, polyethylene, polycarbonate, and the like.

The hollow tube 3 may be calibrated (not shown) for indicating a distance from a balloon 1 side end of the probe 10. Further, as shown in FIGs. 1 and 2, concavities and convexities 3a may be formed on the hollow tube 3 to improve the handling property.

As shown in FIG. 3, in the probe 10 of this embodiment, the hard ring 8 is mounted on a portion 1c between an inflated portion 1a and the attaching portion 1b of the balloon 1. The hard ring 8 prevents the balloon 1 from being pressed under lip pressure, incisive pressure, and the like when the balloon 1 is inserted into an oral cavity, resulting in no adverse effect on a result of pressure measurement. Further, when the balloon 1 is put in an oral cavity, the hard ring 8 is positioned so as to be sandwiched between lips or teeth. As a result, stable pressure measurement and rehabilitation become possible. It is desirable that the arranged position, the length, and the like of the hard ring 8 in the probe 10 are adjusted so as to obtain the above-described effects sufficiently. The hard ring 8 may be sufficiently hard so as not to be deformed under incisive pressure and the like.

As shown in FIG. 2, the hard ring 8 preferably has a flat, e.g., oval outer shape when seen in a longitudinal direction so that the hard ring 8 can be seen internally. When the hard ring 8 is flat, the probe 10 can be held with teeth or the like more stably than when the hard ring 8 is a substantially round tube, for example. As a result, stable pressure measurement can be performed, and rehabilitation can proceed easily.

There is no specific limitation on the material of the hard ring 8. For example, polycarbonate, polypropylene, polyethylene, and the like are preferable.

The balloon 1 preferably is made of an elastic material such as, for example, a natural rubber, a synthetic rubber, a silicone rubber, and the like. A flexible material such as nonrigid plastic also can be used. When an elastic material is used for the balloon 1, the elastic material may be formed into a predetermined shape by the same forming technique as for a medical balloon or a balloon. When a flexible material is used for the balloon 1, a film made of the flexible material may be formed like a bag.

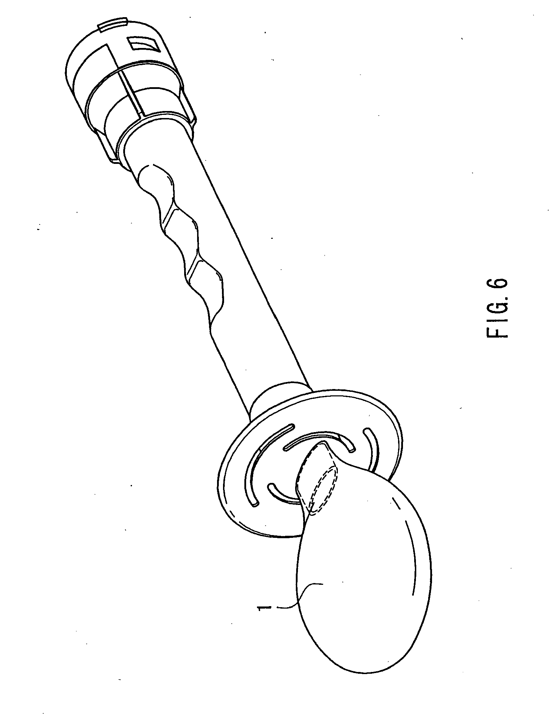

As shown in FIGs. 1 and 2, in a state where the balloon 1 is inflated, the inflated portion 1a may have a substantially spherical shape, for example. The shape also may be flat as shown in FIG. 6. When the inflated portion of the balloon 1 has a spherical shape, the balloon 1 is less stable on a tongue. Thus, when the balloon 1 with the spherical inflated portion 1a is used for the measurement of lingual pressure of a patient whose tongue is removed partially, for example, there may be an adverse effect such as decreased accuracy on a result of pressure measurement. On the other hand, when the inflated portion of the balloon 1 has a flat shape, the balloon becomes more stable on a tongue, resulting in stable pressure measurement and easy rehabilitation.

Further, the balloon 1 may have an anti-slip property such as a satin-finished surface.

As shown in FIGs. 1 and 2, the probe 10 preferably further includes the bumper 14 arranged on an outer periphery of the hollow tube 3. The bumper 14 can prevent the probe 10 from being inserted excessively into an oral cavity, resulting in improved safety in use. Further, the bumper 14 also may have a function of helping positioning for the measurement of lingual pressure.

There is no specific limitation on the shape of the bumper 14. For example, a ring-shaped bumper including a flat surface orthogonal to the longitudinal direction of the hard ring 8 and the probe 10 is preferable. The reason for this, for example, is that it is possible to suppress a variation in the arranged position of the balloon 1 during pressure measurement by keeping lips or the like pressed against the flat surface of the bumper 14 during pressure measurement. Further, the bumper 14 preferably has a through hole 14a that penetrates in its thickness direction. The reason for this is that in the event that a patient puts the bumper 14 in his/her oral cavity and catches it in his/her throat, an open airway needs to be established. For this reason, it is preferable to provide a plurality of through holes 14a.

The bumper 14 preferably is made of an elastic material or a flexible material since it may be pressed against lips or the like. For the same reason, the bumper 14 preferably can be bent under pressure applied from the balloon 1 side.

There is no specific limitation on the shape of the through hole 14. For example, the through hole 14 preferably has an arc shape so as to be bent easily under pressure applied from the balloon 1 side.

The bumper 14 may be integrated with the hard ring 8 or the hollow tube 3, for example, or may be independent of the hard ring 8 and the hollow tube 3. When the bumper 14 is independent of the hard ring 8 and the hollow tube 3, the bumper 14 has through holes into which the hard ring 8 and the hollow tube 3 can be inserted, for example. By inserting the hard ring 8 and the hollow tube 3 through the through holes, the bumper 14 is provided on the outer peripheries of the hard ring 8 and the hollow tube 3.

The bumper 14 may be fixed to the hard ring 8 or the hollow tube 3 so as not to be moved, or may be provided so as to be slidable in the longitudinal direction of the probe 10.

In order to connect the balloon 1 and the hollow tube 3 airtightly, instead of the method of using the shrinkage of the shrinkable tube 12 (see FIG. 3), the following methods, for example, also may be used: a method of bonding with an adhesive, a method of tightening the balloon 1 with the bumper 14, a method of tightening the balloon by using a fitting member made of a hard material, and the like. Examples of the hard material include rigid plastic such as polypropylene, polyethylene, polycarbonate, and the like. The fitting member may be independent, or may be formed integrally with the hard ring 8. Further, the balloon 1 may be fixed to either an inner surface or an outer surface of the hollow tube 3.

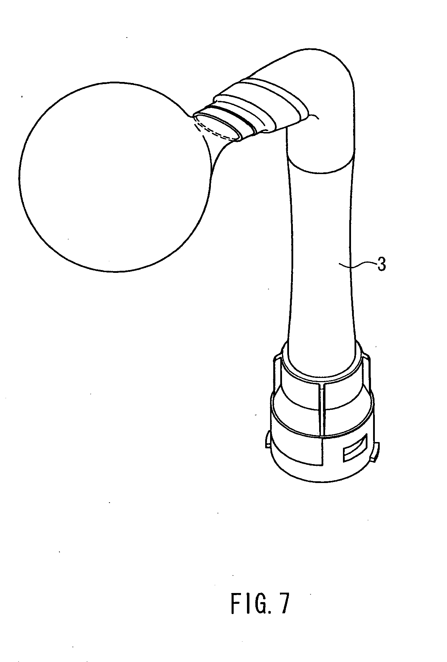

As shown in FIG. 7, the hollow tube 3 may be bent into an L-shape. With the L-shaped hollow tube, it is possible to prevent the probe for measuring oral pressure from being inserted excessively into an oral cavity, resulting in improved safety in use.

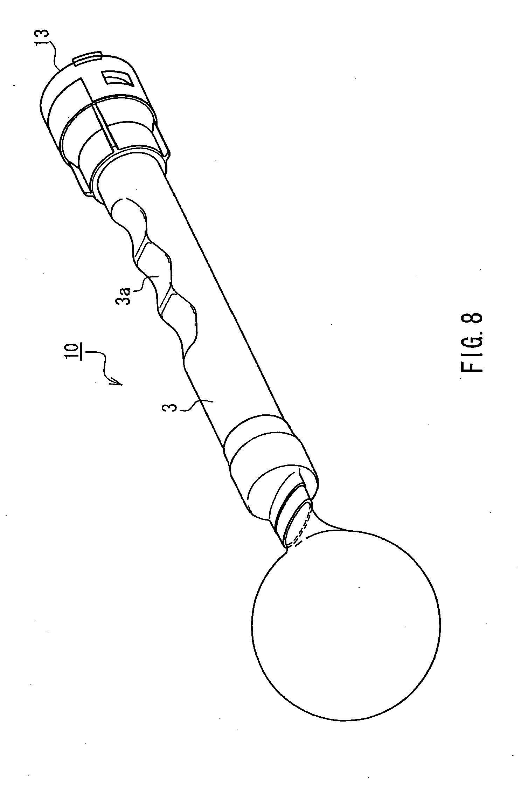

The example of the probe 10 described with reference to FIGs. 1 and 2 includes the bumper 14. However, the probe 10 of this embodiment is not limited thereto, and the bumper 14 may not be provided as shown in FIG. 8.

Further, in the probe 10 of this embodiment described with reference to FIGs. 1 and 2, a cylindrical body having opening portions (the first opening portion and the second opening portion) at its both ends in the longitudinal direction is used as the hollow tube 3. However, the configuration of the hollow tube 3 is not limited thereto. For example, the hollow tube 3 may be a cylindrical body with a bottom that includes in an outer peripheral portion a first opening portion to be connected to the balloon 1 so as to communicate with the inside of the balloon 1, and a second opening portion for fixing the valve.

### (Embodiment 2)

In Embodiment 2, an example of a device for measuring oral pressure according to the present embodiment will be described. FIG. 9 is a conceptual diagram showing an example of the device for measuring oral pressure of this embodiment. In this embodiment, the probe of Embodiment 1 is used as a probe for measuring oral pressure.

In FIG. 9, reference numeral 10 denotes a probe, which is connected to a pressure detecting portion 7 via a main body tube 6. The probe 10 has a first connecting portion 4 (connecting portion). When the first connecting portion 4 and a second connecting portion 5 (connecting portion) of the main body tube 6 are connected, the probe 10 and the main body tube 6 are connected to each other. A selector valve 11 is provided between the main body tube 6 and the pressure detecting portion 7. A pressurizing portion 12 is connected to the main body tube 6 via the selector valve 11. The selector valve 11 is not essential according to the configuration of the pressurizing portion 12, and the pressurizing portion 12 may be connected directly to the main body tube 6.

With the above-described configuration, the inside of the balloon 1 communicates with the pressure detecting portion 7 via the hard ring 8, the hollow tube 3, and the main body tube 6. The pressure detecting portion 7 includes a pressure transducer for converting air pressure into an electric signal, and an amplifier for amplifying the electric signal, and its output is supplied to a display portion 9.

The first connecting portion 4 includes, for example, the cover 16 (see FIG. 1) that covers an upper peripheral portion of the disk-shaped valve 13 to hold the same tightly. The cover 16 includes protruding portions 16a (see FIGs. 1 and 5) that protrude in its radial direction, and the fitting hole 16b (see FIG. 1) at its center. On the other hand, the second connecting portion 5 of the main body tube 6 shown in FIG. 9 includes, for example, a male member (male luer), and a cap member surrounding the male member on an inner surface of which a spiral groove that can be screwed with the protruding portions 16a is formed. The first connecting portion 4 and the second connecting portion 5 are connected airtightly to each other by the frictional force caused by pressing the male member into the fitting hole 16b of the cover, and screwing between the protruding portions 16a and the spiral groove.

As described above, by using, as a system for connecting the probe 10 and the main body tube 6, a luer lock system that establishes connection by the frictional force and screwing, the probe 10 easily can be attached/detached to/from the pressure detecting portion 7. Further, since the probe 10 and the main body tube 6 can be connected attachably/detachably to each other, the probe 10 can be disposable to be exchanged for each measurement of lingual pressure. As a result, in the measurement of lingual pressure, a portion to be put into a mouth always can be renewed sanitarily.

In this embodiment, the main body tube 6 and the probe 10 are connected directly. However, they may be connected indirectly via another tube or the like. Further, the system for connecting the probe 10 and the main body tube 6 is not limited to the luer lock system. For example, a connection system that depends only on the frictional force, or a system that establishes connection by the frictional force and fitting also may be used.

Connection can be established by the frictional force and fitting in the following manner, for example. For example, the second connecting portion 5 of the main body tube 6 includes a male member (for example, a male luer) that can be inserted into the slit, and a cap member surrounding the male member on an inner surface of which cut-away portions that can be fitted with the protruding portions 16a of the cover 16 are formed. After pressing the male member into the fitting hole 16b, the cap member is turned so that the protruding portions 16a are fitted in the cut-away portions, thereby connecting the main body tube 6 and the probe 10 firmly.

The second connecting portion 5 preferably is made of rigid plastic. However, the portions of the main body tube 6 other than the second connecting portion 5 preferably are made of nonrigid plastic such as soft polyvinyl chloride, polybutadiene, soft polypropylene, soft polyethylene, ethylene-vinyl acetate copolymer, and the like in terms of the operability. However, when the main body tube 6 is made of an excessively flexible and thin material, it becomes difficult to perform accurate pressure measurement. On this account, the main body tube 6 preferably is moderately flexible and thick.

The pressure detecting portion 7 is configured so as to convert air pressure into an electric signal using a pressure inlet type strain gauge pressure transducer, for example. The pressure detecting portion 7 may include an amplifier for amplifying the signal before outputting it to the display portion 9. Any other type of pressure transducer also may be used. Further, as the pressure detecting portion 7, a dedicated pressure detector may be designed and manufactured for use, or a pressure detector used for medical purposes such as the measurement of blood pressure also may be used as it is.

The display portion 9 may only display the pressure inside the balloon 1 detected by the pressure detecting portion 7 on its monitor. However, it preferably has a function of recording the detected pressure continuously. As the display portion 9, a digital oscillo-recorder may be used, for example.

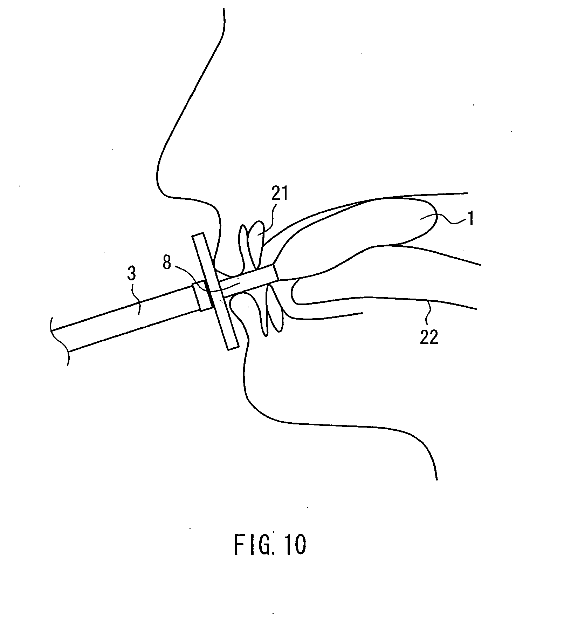

In order to measure lingual pressure, as shown in FIG. 10, the balloon 1 is put into a mouth in a state where the hard ring 8 is positioned so as to be sandwiched between lips or teeth 21. In this state, when a tongue 22 presses the balloon 1 at a maximum pressure, a maximum lingual pressure can be measured. Further, when the balloon 1 is put into a mouth in a state where a liquid is present in the mouth, followed by a swallowing operation, and a pressure change caused by the swallowing operation is monitored continuously, lingual pressure for swallowing can be measured. In this manner, by measuring pressures for various operations, a lingual function of a patient can be analyzed. When the relationship between lingual pressure and a feeding and swallowing operation is elucidated, it is possible to evaluate the feeding and swallowing function. Pressure inside the balloon 1 is not particularly limited, and 10 to 30 kPa, for example, is suitable.

When a patient with an impaired feeding and swallowing function uses the probe 10 alone for repeated training for increased lingual pressure, the feeding and swallowing function may be restored.

In Embodiments 1 and 2, the description has been given of the probe for measuring oral pressure that also can be used as a training tool for restoring an oral function. However, the probe 10 may be used only as a training tool for restoring an oral function. Since the probe 10 allows air to enter and exit the balloon by using a needleless syringe, for example, a patient easily can conduct training for increased lingual pressure at home or the like.

In Embodiments 1 and 2, the description has been given of the measurement of lingual pressure. However, an example of the probe for measuring oral pressure according to the present invention also can be used to measure pressure of hypoglossal muscle, lip pressure, cheek pressure, and the like, as well as lingual pressure. In such a case, it is preferable to change the size of the balloon, the thickness and shape of the tube, and the like depending on the purpose so as to use suitable components.

As described above, in the present invention, the balloon easily can be kept inflated due to the valve. Therefore, according to the present invention, it is possible to provide the probe for measuring oral pressure that easily can be used not only as a probe for measuring lingual pressure and the like but also as a rehabilitation tool for restoring an oral function, and the device for measuring oral pressure using the same. Further, it is also possible to provide the training tool used for restoring an oral function of a patient. Industrial Applicability

The probe for measuring oral pressure according to the present invention also can be used as a training tool for restoring an oral function. Therefore, it is useful for measuring and analyzing functions of a tongue, hypoglossal muscle, a lip, a cheek, and the like, for example, and restoring a lingual function and the like.

## Claims

1. A probe for measuring oral pressure comprising:
a balloon;
a hollow tube that has a first opening portion and a second opening portion and is connected to the balloon so that the first opening potion communicates with an inside of the balloon; and
a port member with a valve connected to the hollow tube so as to block the second opening portion.

2. The probe for measuring oral pressure according to claim 1, wherein the valve has a slit.

3. The probe for measuring oral pressure according to claim 2, wherein a male luer can be inserted into the slit of the valve.

4. The probe for measuring oral pressure according to claim 1, wherein the hollow tube is bent into an L-shape.

5. The probe for measuring oral pressure according to claim 1, wherein the balloon has an inflated portion and an attaching portion to the hollow tube,
the probe for measuring oral pressure further comprising a hard ring mounted on a portion between the inflated portion and the attaching portion of the balloon.

6. The probe for measuring oral pressure according to claim 1, wherein the inflated portion has a flat shape when the balloon is inflated.

7. The probe for measuring oral pressure according to claim 5, wherein the hard ring has a flat outer shape when seen in a longitudinal direction so that the hard ring can be seen internally.

8. The probe for measuring oral pressure according to claim 5, wherein the hard ring has an oval outer shape.

9. The probe for measuring oral pressure according to claim 1, further comprising a bumper arranged on a side of the hollow tube rather than the inflated portion of the balloon.

10. The probe for measuring oral pressure according to claim 9, wherein the bumper is slidable in a longitudinal direction of the probe for measuring oral pressure.

11. The probe for measuring oral pressure according to claim 9, wherein the bumper has an opening portion that penetrates in a thickness direction of the bumper.

12. The probe for measuring oral pressure according to claim 9, wherein the bumper is made of an elastic material or a flexible material.

13. The probe for measuring oral pressure according to claim 9, wherein the bumper can be bent under pressure applied from a balloon side.

14. The probe for measuring oral pressure according to claim 1, wherein the hollow tube is calibrated for indicating a distance from a balloon side end of the probe for measuring oral pressure.

15. A device for measuring oral pressure comprising: the probe for measuring oral pressure according to any one of claims 1 to 14; and a pressure detecting portion that communicates with an inside of the balloon to detect air pressure in the balloon.

16. The device for measuring oral pressure according to claim 15, further comprising a main body tube with two ends, one being connected to the pressure detecting portion and the other being connected to the probe for measuring oral pressure, that allows the inside of the balloon and the pressure detecting portion to communicate with each other,
wherein the hollow tube of the probe for measuring oral pressure and the main body tube have connecting portions that can be connected attachably/detachably to each other.

17. The device for measuring oral pressure according to claim 16, wherein the probe for measuring oral pressure and the main body tube are connected by a luer lock system.

18. A training tool for restoring an oral function comprising:
a balloon;
a hollow tube that has a first opening portion and a second opening portion and is connected to the balloon so that the first opening potion communicates with an inside of the balloon; and
a port member with a valve connected so as to block the second opening portion.
